# FASCICULE DE BREVET EUROPEEN

(11) **EP 3 253 295 B1**
(45) Date de publication et mention de la délivrance du brevet: **13.09.2023**
(21) Numéro de dépôt: 16705251.3
(22) Date de dépôt: 02.02.2016
(51) Int. Cl.: A61B 10/02

(54) **MICRODISPOSITIF POUR LA CAPTURE IN VIVO DE BIOMARQUEURS CELLULAIRES CIRCULANTS**
MIKROVORRICHTUNG ZUR IN-VIVO-ERFASSUNG ZIRKULIERENDER ZELLBIOMARKER
MICRODEVICE FOR THE IN VIVO CAPTURE OF CIRCULATING CALLULAR BIOMARKERS

(30) Priorité: 02.02.2015 FR 1550806
(43) Date de publication de la demande: 13.12.2017
(73) Titulaire: Centre National de la Recherche Scientifique, 75016 Paris (FR); Centre Hospitalier Universitaire De Toulouse, 31059 Toulouse Cedex 9 (FR); Université Paul Sabatier Toulouse III, 31062 Toulouse Cedex 9 (FR)
(72) Inventeur: CERF, Aline, 31650 Saint-Orens de Gameville (FR); SANSON, Sylvain, 31400 Toulouse (FR); CAYRON, Hélène, 31400 Toulouse (FR); JIMENEZ, Alejandro Kayum, 29220 San Cristobal de las Casas, Chiapas (MX); VIEU, Christophe, 31320 Auzeville Tolosane (FR); MALAVAUD, Bernard, 31500 Toulouse (FR)
(74) Mandataire: Gevers & Orès
(86) Numéro de dépôt international: PCT/FR2016/050218
(87) Numéro de publication internationale: WO 2016/124854

(56) Documents cités:
- EP-A1- 2 523 605
- WO-A1-99/23976
- WO-A1-2007/093009
- WO-A1-2013/025531
- WO-A2-99/16382
- WO-A2-2004/096089
- US-A- 4 685 472
- US-A1- 2004 153 118
- US-A1- 2011 244 443
- US-A1- 2014 066 729

## Description

L'invention se situe dans le domaine des dispositifs de capture et de prélèvement de cellules d'intérêt présentes dans la circulation d'un flux biologique tel que le sang et dont l'analyse après capture permet entre autre de porter un diagnostic.

Depuis quelques années, la tendance dans l'identification et le traitement des cancers est à la personnalisation des soins, ce qui passe par l'étude fine des cellules tumorales. Habituellement ces cellules sont prélevées par biopsie. Cependant une biopsie est invasive et des méthodes alternatives ont été mises au point pour l'éviter.

Les tumeurs cancéreuses libèrent dans la circulation sanguine des cellules appelées « Cellules Tumorales Circulantes» ou CTCs ; il est communément admis que ce phénomène apparaît à un stade précoce de la maladie. L'analyse immunohistochimique de ces cellules permet de porter un diagnostic et d'apporter des informations notamment sur l'agressivité du cancer. Les CTCs représentent donc un biomarqueur d'intérêt à tous les temps de la prise en charge de la maladie cancéreuse, diagnostic, pronostic et surveillance.

Or les CTCs sont présentes en concentration extrêmement faible dans le sang des patients atteints de cancer (environ 1/10⁹ cellules sanguines normales) (Nagrath, S., et al.). L'isolation de ces cellules est donc extrêmement difficile. On connait différents procédés pour procéder à cette isolation.

Le document WO 99/16382 A2 divulgue un cathéter de perfusion ayant un écran pour capturer les matériaux emboliques.

Le système CellSearch^{®} est basé sur une immunodétection. Il se fonde sur la présence à la surface des CTCs d'EpCAM, un antigène membranaire spécifique des cellules d'origine épithéliale. Un échantillon de 7,5 ml de sang est centrifugé, puis mis en présence de nanoparticules ferromagnétiques munies à leur surface d'anticorps anti-EpCAM. Les CTCs sont alors séparées des autres cellules par application d'un champ magnétique. L'inconvénient de ce système est double :
- Il utilise un échantillon de sang très limité (7.5 ml ce qui correspond à 0.15 % du volume sanguin total), dans lequel le nombre de CTCs, compte tenu de leur faible concentration, est très faible,
- Il ne permet pas de détecter les CTCs ayant perdu la protéine EpCAM lors de la transition épithélio-mésenchymateuse (EMT) (Small, A.C., et al.) ce qui représente environ 2/3 de la population totale de ces cellules ; d'autre part, il ne permet la détection que de cellules différenciées dont la durée de vie est limitée et qui ne sont pas les plus dangereuses.

On connait d'autres approches pour isoler les CTCs *in vitro* à partir d'un échantillon sanguin, basées sur la taille des CTCs. En particulier, le système ISET (« Isolation by size of Epithelial Tumor Cells ») utilise la filtration d'un échantillon de sang traité (lyse des globules rouges préalable) sur une membrane en polycarbonate micro perforée ; dans ce système, les CTCs sont préalablement rigidifiées par application de paraformaldéhyde de façon à résister à la forte pression qui est appliquée (Williams, A., et al.).

De façon générale, la sensibilité de la détection *in vitro* est réduite du fait du faible volume des échantillons. En effet, étant donné la rareté des CTCs dans le sang, leur présence dans un échantillon de quelques millilitres peut se chiffrer à quelques unités à un stade déjà avancé du cancer. Leur détection à des stades beaucoup plus précoces est de fait quasi-impossible.

De WO 2010/145 824, on connait le système GILUPI^{®} qui permet une détection *in vivo.* Ce système repose sur la fonctionnalisation d'une tige métallique à l'aide d'anticorps anti-EpCAM introduite dans la circulation sanguine. Ce système permet de s'affranchir de la limitation inhérente au faible volume des échantillons, mais elle reste limitée par la méthode de capture immunologique.

L'invention a pour objectif de proposer un dispositif qui permette de s'affranchir des inconvénients liés à l'échantillonnage et aux méthodes immunologiques pour isoler les biomarqueurs cellulaires circulants. A cet effet, elle propose un dispositif de capture de biomarqueurs cellulaires circulant dans un flux biologique animal ou humain, tel que défini dans la revendication 1.

Le dispositif selon l'invention se fonde sur les propriétés physiques des biomarqueurs cellulaires circulant, taille et déformabilité. Les plaquettes ont une dimension de 2 à 4 µm et les globules rouges une dimension d'environ 7 µm ; les globules blancs ont des dimensions variables, de 7 à 25 µm, mais ils sont très déformables, ce qui leur permet de circuler dans les plus petits vaisseaux. Parmi les biomarqueurs cellulaires circulants, les cellules tumorales circulantes, en particulier, ont des dimensions variables, comprises entre 4 et 25 µm, mais ils sont très peu déformables. En conséquence, il est possible de prévoir un moyen de filtration qui retienne les biomarqueurs cellulaires circulants tels que les CTCs tout en laissant passer tous les autres composants du flux biologique dans des conditions *in vivo,* notamment de pression et de vitesse. Le dispositif de capture selon l'invention n'utilise que les propriétés physiques (taille, déformabilité) des biomarqueurs cellulaires, on s'affranchit donc de toute hypothèse sur la présence de protéines membranaires à la surface des CTCs. De préférence, le dispositif ne comprend pas de moyens de capture des biomarqueurs cellulaires basés sur des affinités chimiques des biomarqueurs. Les biomarqueurs cellulaires pénètrent dans le creux du support et sont retenus du fait de leur propriétés physiques par filtration. Cette forme du dispositif de capture permet de limiter le phénomène de recirculation des biomarqueurs cellulaires. D'une part, le dispositif de capture présentant un support creux, il permet d'éviter que les biomarqueurs ne s'échappent latéralement. D'autre part, le dispositif de capture permet également d'éviter que des biomarqueurs ayant pénétré dans le dispositif de capture ne soient entraînés à l'extérieur de ce dernier par des mouvements de flux interne à celui-ci. Le dispositif de capture est notamment prévu pour limiter les tourbillons contrarotatifs.

Des exemples de flux biologiques sont le sang, la lymphe, l'urine...

Des exemples de biomarqueurs cellulaires circulants pour lesquels le dispositif de capture est adapté sont les cellules stromales, épithéliales, les cellules foetales circulantes (CFC), les cellules souches, clusters ou agrégats de cellules tumorales, les cellules tumorales associées à tous types de cancer : cellules tumorales prostatiques, du cancer du sein, du colon,... et les autres types cellulaires: monocytes (macrophages). Le dispositif selon l'invention est donc particulièrement adapté pour le diagnostic de différents types de cancers par le biais de la capture de cellules tumorales circulantes, notamment.

On entend aussi par biomarqueurs cellulaires des éléments infestants de grande taille comme les parasites intra ou extracellulaires ou leurs oeufs. Par exemple, la leishmaniose peut être diagnostiquée par la présence de sa forme infectante, promastigote, d'une taille de 10 µm à 15 µm. De même, la chistosomiase peut être diagnostiquée par la présence de ses oeufs d'une taille de 70 µm à 200 µm.

De manière préférée, les biomarqueurs cellulaires sont des cellules eucaryotes. Ces cellules peuvent être individuelles ou sous forme d'agrégats.

Le diagnostic d'autres maladies pourrait être envisagé suite à la capture d'autres biomarqueurs cellulaires circulants pouvant notamment présenter des anomalies. Le diagnostic d'états physiologiques autres que des maladies ou la prévention de maladies peut également être envisagé par la capture de biomarqueurs cellulaires circulants.

Le dispositif de capture selon l'invention est apte à être utilisé in vivo. En particulier, il peut être prévu pour être placé dans le flux de la circulation sanguine. Le moyen de filtration est donc associé à un support pour le rigidifier et/ou pour le retenir au sein du flux biologique et permettre sa récupération. Le dispositif de capture peut être un dispositif de capture in vivo, in vitro ou ex vivo. De préférence, le dispositif de capture est un dispositif in vivo. Toutefois, il n'est pas exclu qu'il puisse être utilisé ex vivo ou in vitro.

Le dispositif de capture peut comporter l'une ou l'autre des caractéristiques suivantes, seule ou en combinaison.

Avantageusement, le dispositif de capture est apte à filtrer des biomarqueurs cellulaires lorsque la pression de flux biologique auquel il est soumis est comprise entre 9 et 16 mmHg. Ces pressions sont typiquement les pressions qui se retrouvent dans les flux biologiques in vivo, au niveau des veines périphériques (Munis, J. R. et al.).

Avantageusement, le moyen de filtration comporte au moins une ouverture traversante dont les dimensions sont adaptées pour retenir les biomarqueurs cellulaires en fonction de leur taille et de leur déformabilité dans le flux sanguin.

Les dimensions sont par exemple la profondeur et au moins une dimension transversale.

Avantageusement, au moins une ouverture peut être sensiblement circulaire ou sensiblement ovale ou sensiblement polygonale ou prendre la forme d'une fente.

L'invention n'est pas limitée par la forme des ouvertures, pour autant bien sûr qu'elles soient traversantes. La forme et la dimension des ouvertures peuvent varier au sein de l'épaisseur du moyen de filtration afin de permettre la capture de différents types de biomarqueurs cellulaires à différentes épaisseurs. La paroi de ces ouvertures peut former un cône tronqué, mais de préférence elle forme un cylindre, c'est-à-dire comportant des génératrices parallèles.

Le moyen de filtration peut comporter seulement une ouverture, par exemple sous la forme d'une fente, rectiligne ou courbe, dont la dimension minimale est choisie pour pouvoir retenir les biomarqueurs cellulaires considérés ou plusieurs ouvertures, également ou inégalement réparties sur le moyen de filtration, de même forme et dimension ou de forme et/ou de dimensions différentes.

Selon un mode de réalisation préféré, chaque ouverture présente une dimension transversale, de préférence sa dimension transversale minimale, comprise entre 1 µm et 200 µm, plus préférablement entre 1 µm et 100µm, encore plus préférablement entre 5µm et 100µm. En particulier, chaque ouverture présente une dimension transversale comprise de préférence entre 1µm et 25µm pour des biomarqueurs cellulaires de type CTC, plus préférablement entre 5µm et 25µm, encore plus préférablement entre 12 µm et 25 µm, le diamètre optimum étant dans ce cas voisin de 12 µm.

De préférence, chaque ouverture traversante du moyen de filtration est circulaire. La dimension transversale correspond alors au diamètre de l'ouverture.

Le support est une pièce creuse ouverte à une extrémité et fermée à une autre extrémité par le moyen de filtration. De préférence, le support s'étend selon une direction longitudinale. L'ouverture du support peut alors être à une extrémité longitudinale et le moyen de filtration à l'extrémité longitudinale opposée.

Avantageusement, le moyen de filtration peut être plan.

L'invention n'est pas limitée non plus par la forme du moyen de filtration, mais il est de préférence plan.

Selon un mode de réalisation, le support possède une symétrie de révolution autour d'un axe. Le support possède une symétrie de révolution totale autour d'un axe ou une symétrie de révolution partielle et être tronqué selon un plan parallèle à l'axe. En utilisant un dispositif avec une symétrie de révolution, on diminue la perturbation de l'écoulement du flux biologique, lorsque le dispositif est placé avec son axe de révolution dans la direction principale du flux biologique. Ce type de forme permet notamment de diminuer le risque de thrombose lorsque le dispositif est utilisé *in vivo.* La partie tronquée permet de mieux fixer le dispositif de capture sur un moyen d'introduction. Elle est de préférence destinée à être en contact avec le moyen d'introduction. L'axe de révolution est de préférence la direction longitudinale selon laquelle s'étend le support.

Le dispositif de capture peut par exemple prendre la forme d'un cylindre, d'un cône ou d'une paraboloïde tronqué ou non. moyen de filtration forme le fond du support.

Le dispositif possède une symétrie de révolution autour d'un axe. Le dispositif de capture peut par exemple prendre la forme d'un cylindre ou d'un cône, ou d'un cylindre surmonté d'un cône ou d'une calotte sphérique.

Dans un mode préféré de réalisation, le dispositif peut prendre la forme d'un cylindre creux, tronqué selon un plan parallèle à l'axe du cylindre et le moyen de filtration peut former le fond du cylindre tronqué.

Le support peut comporter au moins une ouverture traversante sensiblement circulaire ou sensiblement ovale ou sensiblement polygonale ou prend la forme d'une fente. De préférence, le support ne comprend pas d'ouverture traversante dont les dimensions sont prévues pour retenir les biomarqueurs cellulaires.

L'invention n'est pas limitée non plus par la présence ou l'absence d'ouvertures traversantes dans le support, leur forme ou leur nombre, pouvant être différents de ceux des ouvertures présentes sur le moyen de filtration.

Selon un mode de réalisation, le support et le moyen de filtration forment une seule pièce.

Avantageusement, le dispositif, moyen de filtration et support peut être réalisé en une résine photosensible.

Le dispositif, moyen de filtration et support, peut être réalisé par lithographie laser tridimensionnelle ou photolithographie conventionnelle: on polymérise la résine à l'endroit où elle est irradiée, puis la résine non polymérisée est dissoute.

Avantageusement, le dispositif peut être réalisé en un matériau biocompatible ou il peut être recouvert d'un matériau biocompatible, par exemple un polymère tel que le parylène ou il peut être recouvert d'une couche métallique biocompatible (Au, Ti,...). Ce matériau biocompatible peut également prendre la forme d'un gel compatible avec la culture cellulaire.

Avantageusement, le dispositif de capture peut être fonctionnalisé à l'aide d'un composé anti-thrombotique tel que l'héparine de façon à éviter le risque de thrombose ou de coagulation. Le dispositif peut notamment être exposé à divers traitements de surface visant à modifier ses propriétés d'adsorption vis à vis des entités biologiques circulantes. Ces traitements peuvent être des traitements plasmas permettant soit le contrôle de la tension de surface, de la réactivité chimique de surface ou de la rugosité de surface mais également des traitements chimiques de greffage ou de fonctionnalisation. Avantageusement, le dispositif de capture peut être fonctionnalisé à l'aide d'un composé anti-thrombique.

L'invention porte également sur un dispositif de prélèvement de biomarqueurs cellulaires, comportant au moins un dispositif de capture de biomarqueurs selon l'invention fixé sur un moyen d'introduction du dispositif de capture dans un vaisseau de flux biologique.

Le dispositif de capture est associé à un moyen d'introduction afin de l'introduire facilement au sein d'un vaisseau de flux biologique, notamment un vaisseau sanguin, une artère ou de préférence une veine, ou un vaisseau lymphatique. Le dispositif de capture peut être fixé directement ou moyen d'introduction ou par l'intermédiaire d'une pièce telle qu'un plot. Bien entendu, le dispositif de prélèvement peut comporter plusieurs dispositifs de capture, pouvant être différents les uns des autres, notamment en ce qui concerne la forme et le diamètre des ouvertures.

Dans une variante, le moyen d'introduction du dispositif de capture dans la circulation sanguine peut former le support.

Dans cette variante, il y a identité entre le support du moyen de filtration et le moyen d'introduction.

Avantageusement, le moyen d'introduction du dispositif de capture dans la circulation sanguine peut comporter une languette.

Une fine languette, par exemple métallique, peut servir de moyen d'introduction du dispositif de capture dans le vaisseau sanguin.

Avantageusement, la languette peut comporter un alliage métallique ou un matériau composite à base de résine et de fibres.

Il peut s'agir d'un acier inoxydable ou d'un alliage de Nickel et de Titane tel que le Nitinol, connu pour ses propriétés de biocompatibilité et sa flexibilité. En effet, la languette doit être en un matériau suffisamment flexible pour pouvoir s'insérer selon l'axe longitudinal du vaisseau sanguin.

Dans le cas d'une languette en acier inoxydable ou en matériau composite, celle-ci peut être recouverte d'un matériau biocompatible tel que le parylène ou d'une couche métallique biocompatible (Au, Ti,...). Le dépôt de matériau biocompatible permet également d'apporter de la rigidité à la languette ou d'améliorer sa tenue mécanique.

Avantageusement, le moyen d'introduction peut comporter un premier moyen de protection du moyen de filtration prévu pour éviter le détachement du moyen de filtration.

Il est avantageux de protéger le moyen de filtration du dispositif de capture, voire le dispositif de capture dans son ensemble, afin d'éviter son détachement du moyen d'introduction lors de la manoeuvre d'introduction ou de retrait. A cet effet le dispositif de prélèvement peut comporter un premier moyen de protection. Une fonction de ce premier moyen de protection peut aussi être également de centrer le dispositif de prélèvement par rapport au flux dans le vaisseau sanguin, et aussi par rapport au cathéter lorsqu'il est introduit dans un cathéter.

Avantageusement, le premier moyen de protection peut comporter au moins un plot fixé sur le moyen d'introduction.

Le moyen d'introduction peut par exemple comporter deux plots encadrant le ou les dispositifs de capture et suffisamment éloignés pour ne pas perturber le flux sanguin.

Avantageusement, le moyen d'introduction peut comporter un second moyen de protection prévu pour protéger un vaisseau sanguin dans lequel il est amené à être introduit.

Il est avantageux de protéger les parois du vaisseau sanguin du corps étranger que représente le dispositif de prélèvement.

Avantageusement, le dispositif de prélèvement peut comporter une pièce terminale fixée à l'extrémité du moyen d'introduction et formant un premier et/ou un second moyen de protection.

Avantageusement, la pièce terminale peut comporter au moins une arche.

Par arche, on entend une pièce allongée courbe comportant une première et une seconde extrémité, pouvant être reliée à la languette par au moins une de ses extrémités.

Lorsque la pièce terminale est fixée à l'extrémité du dispositif de prélèvement, l'au moins une arche prend appui de façon non agressive sur la paroi du vaisseau et centre le dispositif à l'intérieur du vaisseau et du cathéter lorsqu'un cathéter est utilisé. L'au moins une arche peut également être prévue pour protéger le dispositif de capture.

Dans une autre variante, le moyen d'introduction peut comporter au voisinage de son extrémité au moins une arche longitudinale formant un premier et/ou un second moyen de protection.

Avantageusement, le dispositif de prélèvement peut être recouvert de matériau biocompatible tel que le parylène.

Avantageusement, le dispositif de prélèvement peut être fonctionnalisé à l'aide d'un composé anti-thrombotique, par exemple l'héparine.

Avantageusement, le dispositif de prélèvement peut comporter un moyen de préhension de façon à le rendre apte à être utilisé dans un cathéter.

Le dispositif de prélèvement peut comporter un « stopper », c'est-à-dire moyen de préhension tel qu'un un bouchon en plastique, placé sur l'extrémité distale du moyen d'introduction, facilitant la préhension du dispositif et servant de butée lors de l'introduction du dispositif dans le cathéter. On appelle extrémité distale du dispositif de prélèvement l'extrémité qui est à l'opposé de celle qui comporte le dispositif de capture.

Avantageusement, le dispositif de prélèvement peut être inséré dans un cathéter.

L'invention concerne également un ensemble de prélèvement de biomarqueurs cellulaires comprenant un cathéter et un dispositif de prélèvement selon l'invention, ledit dispositif étant inséré dans le cathéter.

Un cathéter de perfusion, éventuellement fonctionnalisé grâce à un composé anti-thrombotique tel que l'héparine, guide et protège avantageusement le dispositif de prélèvement. Le cathéter est introduit dans le vaisseau sanguin, le dispositif de prélèvement est rentré à l'intérieur du cathéter, puis le dispositif de prélèvement est poussé hors du cathéter pour être placé dans le flux sanguin. A l'issue du prélèvement, la manoeuvre inverse est réalisée : rentrée du dispositif de prélèvement dans le cathéter, retrait du cathéter. Les cellules capturées peuvent ensuite être soit comptées, marquées immunologiquement et mises en culture directement sur le dispositif de capture, soit facilement récupérées pour numération, immunomarquage et remise en culture in vitro.

Les avantages du dispositif selon l'invention sont les suivants :
- Il est apte à être utilisé in vivo. Typiquement, ses dimensions permettent son introduction dans un canal biologique tel qu'un vaisseau sanguin.
- Il n'utilise que les propriétés physiques (taille, déformabilité) des biomarqueurs cellulaires, on peut donc s'affranchir de la présence réelle ou supposée de protéines membranaires spécifiques à la surface des biomarqueurs cellulaires,
- La circulation sanguine peut être utilisée directement comme système fluidique,
- Le volume de sang analysé est bien plus important ; le dispositif peut analyser 100 ml de sang en 5 minutes, soit un volume 10 à 100 fois supérieur à celui analysé par les systèmes qui utilisent un échantillon,
- Le nombre de captures peut être augmenté en augmentant le temps de prélèvement et en prévoyant un dispositif comportant plusieurs dispositifs de capture,
- Il est applicable à n'importe quel type de biomarqueur cellulaire circulant précédemment cité en adaptant sa géométrie, notamment la taille des ouvertures.

Il est également divulguée une méthode pour détecter une maladie ou un état physiologique chez un patient comprenant une étape de capture d'un biomarqueur cellulaire par le dispositif de capture selon l'invention et une étape d'analyse des biomarqueurs cellulaires capturés.

L'étape de capture du biomarqueur cellulaire peut être réalisée in vivo, in vitro ou ex vivo.

L'étape d'analyse peut être une étape d'analyse quantitative ou qualitative. Par exemple, une étape de détermination peut être une étape pour déterminer si les biomarqueurs cellulaires sont des cellules cancéreuses.

L'étape d'analyse peut comprendre une étape de remise en culture des biomarqueurs cellulaires.

L'invention concerne un dispositif de capture de biomarqueurs cellulaires selon l'invention pour une utilisation à des fins diagnostiques, notamment à des fins de diagnostic du cancer.

Il est également divulguée une méthode pour détecter une maladie ou un état physiologique chez un patient comprenant une étape de capture d'un biomarqueur cellulaire par le dispositif de prélèvement selon l'invention et une étape de d'analyse des biomarqueurs cellulaires capturés

L'invention concerne également un dispositif de prélèvement de biomarqueurs cellulaires selon l'invention pour une utilisation à des fins diagnostiques, notamment à des fins de diagnostic du cancer.

Il est également divulguée une méthode pour détecter une maladie ou un état physiologique chez un patient comprenant une étape de capture d'un biomarqueur cellulaire par l'ensemble de prélèvement selon l'invention et une étape d'analyse des biomarqueurs cellulaires capturés.

L'invention concerne également un ensemble de prélèvement de biomarqueurs cellulaires selon l'invention pour une utilisation à des fins diagnostiques, notamment à des fins de diagnostic du cancer.

Il est également divulguée une méthode pour traiter une maladie chez un patient comprenant une étape de capture d'un biomarqueur cellulaire par le dispositif de capture selon l'invention.

L'invention concerne un dispositif de capture de biomarqueurs cellulaires selon l'invention pour une utilisation à des fins thérapeutiques, notamment à des fins de thérapie du cancer.

Il est également divulguée une méthode pour traiter une maladie chez un patient comprenant une étape de capture d'un biomarqueur cellulaire par le dispositif de prélèvement selon l'invention.

L'invention concerne également un dispositif de prélèvement de biomarqueurs cellulaires selon l'invention pour une utilisation à des fins thérapeutiques, notamment à des fins de thérapie du cancer.

Il est également divulguée une méthode pour traiter une maladie chez un patient comprenant une étape de capture d'un biomarqueur cellulaire par l'ensemble de prélèvement selon l'invention.

L'invention concerne également un ensemble de prélèvement de biomarqueurs cellulaires selon l'invention pour une utilisation à des fins thérapeutiques, notamment à des fins de thérapie du cancer.

Des modes de réalisation et des variantes seront décrits ci-après, à titre d'exemples non limitatifs, avec référence aux dessins annexés dans lesquels :
- La figure 1 représente en perspective un dispositif de capture, la figure 1A étant une vue agrandie d'une partie de ce dispositif,
- Les figures 2A et 2B représentent en perspective des variantes de moyens de filtration, la figure 2C représente en perspective une variante de dispositif de capture,
- Les figures 3A et 3B représentent les extrémités de deux modes de réalisation d'un dispositif de prélèvement,
- La figure 4 représente l'extrémité d'un dispositif de prélèvement comportant deux moyens de protection,
- Les figures 5A et 5B représentent l'extrémité d'un dispositif de prélèvement et une pièce terminale de protection, la figure 5C est une vue agrandie de la figure 5B,
- Les figures 6A et 6B représentent l'extrémité d'un dispositif de prélèvement comportant un moyen de protection et de centrage dans deux variantes,
- Les figures 7A à 7E représentent des variantes de dispositif de capture.

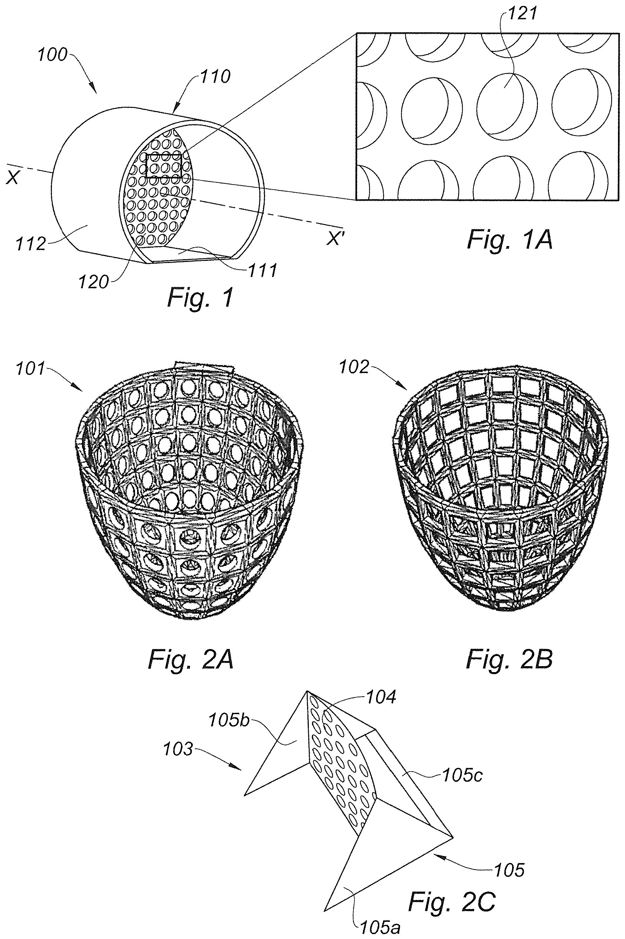

Le dispositif de capture 100 illustré en figure 1 comporte un support cylindrique 110 tronqué selon un plan parallèle à l'axe X-X' du cylindre, c'est-à-dire que ce support comporte une partie plane 111 et une partie arrondie 112. Comme il sera vu plus loin, la partie plane 111 est utile pour la fixation du dispositif sur un moyen d'introduction ; cependant le dispositif pourrait être fixé par une génératrice d'un cylindre non tronqué ou sur les arêtes de la génératrice du cylindre tronqué.

A une première extrémité le cylindre est fermé par un fond plan 120 et il est ouvert à son autre extrémité. Le fond 120 constitue le moyen de filtration du dispositif 100 sous la forme d'une membrane filtre. Comme il est visible sur la figure 1A, la membrane filtre 120 comporte une série d'ouvertures traversantes 121 de forme cylindrique de section circulaire de même diamètre.

Le dispositif de capture 100 est prévu pour être placé dans la circulation sanguine, l'ouverture étant orientée vers l'amont de la circulation de façon à ce que les biomarqueurs recherchés pénètrent dans le dispositif de capture et soient retenus par le filtre 120. Il importe que ces biomarqueurs ne s'échappent pas du dispositif de capture une fois qu'ils y sont entrés. Or dans certaines configurations, les simulations numériques ont montré qu'il pouvait exister des boucles de remise en circulation à l'intérieur du dispositif et que les biomarqueurs pouvaient s'en échapper. Dans le cas du dispositif cylindrique illustré en figure 1, ce phénomène se produit lorsque le support 110 comporte les mêmes ouvertures 121 que le filtre 120. Dans le mode de réalisation préféré illustré en figure 1, la partie arrondie 112 du support ne comporte pas d'ouverture et permet de garder un flux substantiellement laminaire lors de la traversée du dispositif par le flux sanguin.

Les figures 2A, 2B et 2C illustrent d'autres modes de réalisation du dispositif de capture. En figures 2A et 2B, les dispositifs 101, 102 comportent un moyen de filtration qui prend la forme d'un paraboloïde de révolution, le support du moyen de filtration dans ce cas étant constitué par un moyen d'introduction tel qu'une languette. Le dispositif 101 comporte des ouvertures circulaires, le dispositif 102 comporte des ouvertures polygonales. Ces dispositifs peuvent être tronqués ou munis d'une base ou socle pour faciliter leur fixation sur un moyen d'introduction comme mentionné précédemment en référence à la figure 1.

La figure 2C illustre un dispositif de capture 103 comportant un filtre plan 104 maintenu par un support 105 comprenant deux montants 105a, 105b reliés par une traverse 105c parallèle au plan du filtre 104.

Les dimensions du dispositif sont adaptées à sa mise en oeuvre dans un vaisseau sanguin ; il ne doit pas perturber la circulation sanguine afin de ne pas créer de risque de thrombose notamment.

Dans le cas du dispositif 100 illustré en figure 1, les dimensions sont les suivantes :
- Diamètre compris entre 50 µm et 3 mm, de préférence entre 50µm et 1,7mm, plus préférablement voisin de 200 µm, selon un mode de réalisation, le diamètre du dispositif est compris entre 0,5% et 15% du diamètre du canal dans lequel le dispositif est introduit, de préférence entre 0,5% et 5% en particulier dans le cas où le dispositif de capture est destiné à être placé dans la veine basilique,
- Longueur comprise entre 50 µm et 3 mm, de préférence voisine de 150 µm, de préférence supérieure à la somme de l'épaisseur de la paroi du dispositif et du diamètre du biomarqueur cellulaire destiné à être capturé,
- Diamètre des ouvertures compris entre 1µm et 200µm, de préférence entre 1 µm et 100 µm, encore plus préférablement entre 5µm et 100µm d'une façon générale pour les biomarqueurs cellulaires cités précédemment et entre 1 µm et 25µm, plus préférablement entre 5µm et 25µm, encore plus préférablement entre 12µm et 25µm pour les CTCs; de façon surprenante, et à titre d'exemple non limitatif, on a trouvé que pour les cellules tumorales de lignées prostatiques PC3 de taille comprise entre 12 µm et 25 µm, le dispositif de capture était très sensible au diamètre des ouvertures, le diamètre optimum étant dans ce cas voisin de 12 µm ;
- Lorsque l'ouverture prend la forme d'une fente, sa dimension transversale est comprise entre 1µm et 200µm, de préférence entre 1 µm et 100 µm, encore plus préférablement entre 5µm et 100µm, de préférence comprise entre 1 µm et 25µm, plus préférablement entre 5µm et 25µm, encore plus préférablement entre 12µm et 25µm, de préférence voisine de 12 µm,
- Les ouvertures peuvent être de forme tronconique, le diamètre variant de 1µm et 200µm, de préférence de 1 µm et 100 µm, encore plus préférablement de 5µm et 100µm environ pour les biomarqueurs cellulaires, de préférence entre 1 µm et 25µm, plus préférablement entre 5µm et 25µm, encore plus préférablement entre 12µm et 25µm, d'environ 15 µm à environ 8 µm pour les CTCs, de façon à retenir les biomarqueurs cellulaires et plus particulièrement les CTCs à l'intérieur des ouvertures,
- Epaisseur de la paroi du support et de la membrane filtre comprise entre 5 µm et 20 µm, de préférence voisine de 9 µm ; de préférence l'épaisseur de la membrane filtre est voisine de 6µm et celle de la paroi voisine de 10µm pour augmenter la tenue mécanique du dispositif de capture,
- Dans le cas d'une membrane filtre plane et rectangulaire telle qu'illustrée en figure 2C, des dimensions caractéristiques des montants seront compris entre 50 µm et 1,5 mm.

Le dispositif de capture peut être réalisé à partir d'une résine photosensible travaillée par lithographie laser à 3 dimensions ou toute autre technique de lithographie adaptée aux dimensions du dispositif.

En ce qui concerne la biocompatibilité, l'invention propose deux options :
- Soit la résine utilisée est biocompatible,
- Soit le dispositif une fois fabriqué est recouvert d'un film de biocompatibilité tel que le parylène ou en métal biocompatible tel que Au, Ti....

Le dispositif de capture de l'invention n'est pas limité par la description qui précède. Notamment :
- Le plan de troncage pourrait être angulairement décalé par rapport à l'axe X-X',
- Le support pourrait présenter une autre forme que cylindrique, par exemple conique, sphérique, tronquée ou non tronquée par un plan,
- Lorsque le support ne possède pas d'axe de révolution, la forme du support n'est pas limitée à l'utilisation de deux montants et d'une traverse,
- La membrane filtre pourrait être placée selon une section du cylindre et non en constituer le fond, c'est-à-dire que le support 110 pourrait comporter une partie en aval du flux sanguin,
- Le fond 120 du cylindre, formant la membrane filtre du dispositif 100, pourrait ne pas être plan, mais par exemple prendre la forme d'une calotte hémisphérique ou d'un cône.

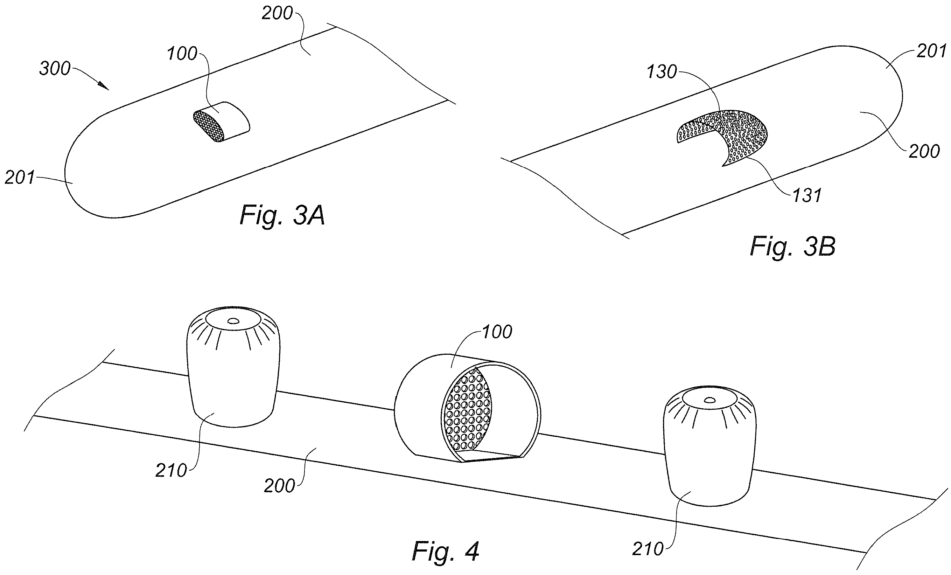

La figure 3A illustre un dispositif de prélèvement 300 comportant un dispositif de capture 100 fixé sur un moyen d'introduction, ici une languette 200, par la partie plane 111 du support 110, au voisinage de l'extrémité 201 de la languette. Il peut être fixé par soudure ou dépôt métallique ou utilisation d'une colle ou résine biocompatibles.

La languette 200 est en Nitinol, qui est un alliage de Nickel et de Titane et qui a la propriété d'être biocompatible et flexible ce qui permet de faciliter son introduction dans le vaisseau sanguin. Dans le cas où elle serait constituée d'un autre matériau, non biocompatible, elle serait recouverte, à un stade de sa fabrication, d'un film de matériau biocompatible tel que le parylène.

La largeur de la languette est comprise entre 300 µm et 3mm, préférablement entre 300µm et 1,5 mm, de préférence voisine de 900 µm, de façon à être compatible avec le diamètre interne des cathéters dans lesquels la languette peut être insérée. Dans le cas d'une languette en Nitinol, son épaisseur est comprise entre 50 µm et 76 µm. Une fois découpée, elle fait quelques centimètres de longueur, par exemple 5 cm, mais la longueur effectivement introduite dans la circulation sanguine est limitée à 1 cm ou 2 cm.

La figure 3B illustre un mode de réalisation alternative ne faisant pas partie de l'objet revendiqué dans lequel le support de la membrane 130 est constitué par la languette 200. La membrane filtre est fixée sur la languette 200 par un bord 131 de telle sorte à présenter une ouverture destinée à être orientée vers l'amont du flux sanguin. La membrane filtre est configurée de telle sorte à ne pas perturber le flux sanguin.

Un risque associé au dispositif de prélèvement 300 est que le ou les dispositifs de capture 100 se détachent de la languette 200 et entrent dans la circulation sanguine, par exemple au moment de l'introduction ou du retrait du dispositif de prélèvement. A cet effet, tel qu'illustré en figure 4, la languette 200 comporte des premiers moyens de protection sous la forme de plots 210, fixés sur la languette 200 de part et d'autre d'un ou plusieurs dispositifs de capture 100 dans le sens longitudinal. Les plots illustrés en figure 4 présentent une forme de "tonneau" à sommet arrondi, mais ils pourraient présenter une forme cylindrique, hémisphérique, tronconique... pour autant qu'ils se présentent sous la forme d'un corps relativement massif à l'échelle du dispositif et de formes arrondies pour ne pas perturber la circulation sanguine ni blesser la paroi du vaisseau.

Les plots 210 sont suffisamment proches du dispositif de capture 100 pour lui procurer une protection mais assez éloignés, par exemple séparés d'environ 1 mm, pour ne pas perturber le flux sanguin autour du dispositif de capture 100.

Les plots 210 collés sur la languette peuvent être avantageusement constitués de résine et fabriqués avec le même procédé de fabrication que le dispositif de capture 100, sans que ceci soit limitatif.

Un autre avantage des plots 210 est qu'ils peuvent participer à la fonction de centrage du dispositif de prélèvement dans le vaisseau et/ou dans un cathéter.

Un autre risque associé au dispositif de prélèvement 300, compte tenu notamment du temps de capture de plusieurs minutes, est celui d'endommager la paroi du vaisseau dans lequel il est inséré. A cet effet l'invention propose deux seconds moyens de protection :

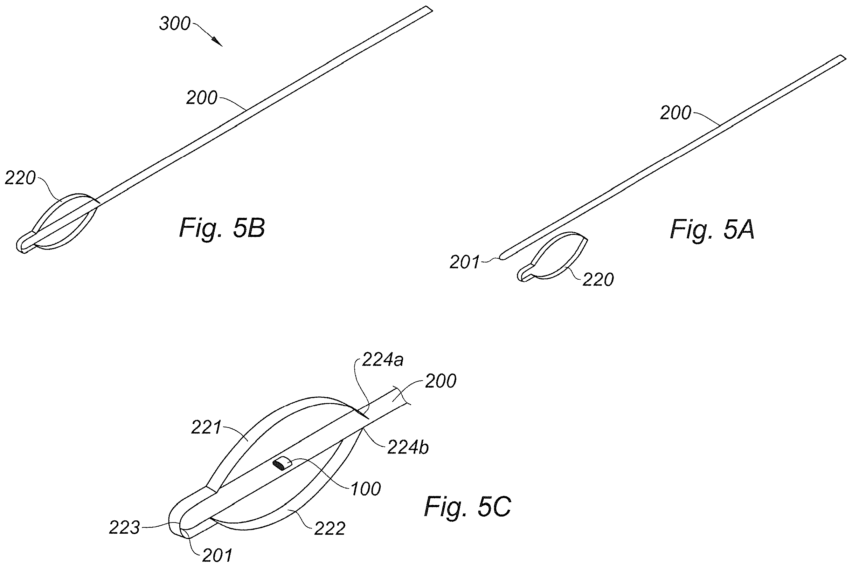

- Les figures 5B et 5C illustrent une languette 200 munie d'une pièce terminale 220 prévue pour se fixer sur la languette, au voisinage de son extrémité ; cette partie terminale 220 forme deux arches 221, 222 réunies à une première extrémité commune 223, et dont les secondes extrémités 224a, 224b sont libres ; cette pièce terminale 220 se fixe sur la languette 200 d'une part en réunissant l'extrémité commune 223 et l'extrémité 201 de la languette, d'autre part en fixant les secondes extrémités 224a, 224b sur des faces opposées de la languette ; ces arches 221, 222 prennent appui de façon non agressive sur la paroi du vaisseau et le protègent ; elles protègent également de l'arrachement le dispositif de capture 100 fixé sur la languette 200 entre les premières et secondes extrémités de ces arches ;

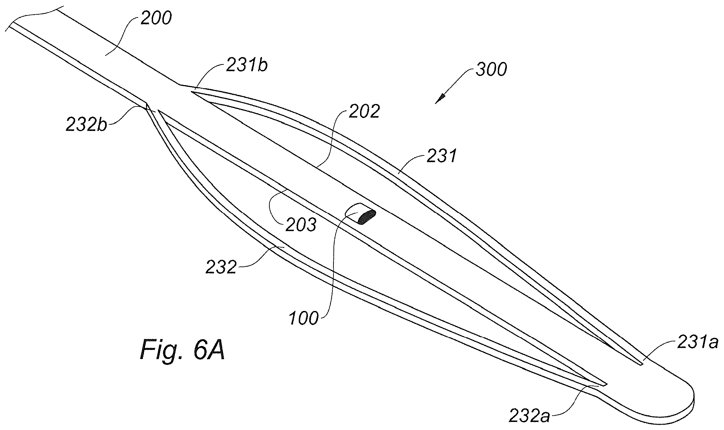

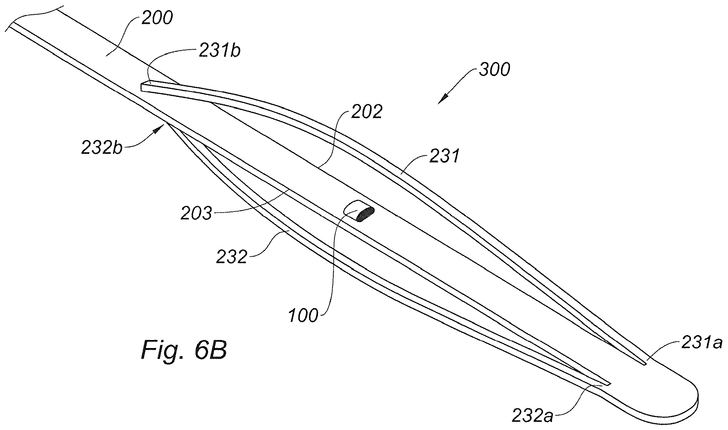

- La figure 6A illustre une languette 200 dont une lamelle 231, 232 est détachée de chacun des bords 202, 203 de la languette 200 et forme une arche, les extrémités 231a, 231b, 232a, 232b de ces lamelles restant attachées à la languette ; les lamelles 231, 232 s'étendent de part et d'autre du plan de la languette 200 de sorte que, comme dans le cas de la figure 5, ces arches protègent le vaisseau et centrent le dispositif de prélèvement 300 dans le vaisseau. Dans la variante de la figure 6B, seules les extrémités aval 231a, 232a restent attachées aux bords 202, 203 de la languette 200 ; les lamelles 231, 232 sont légèrement vrillées de telle sorte que les extrémités amont 231b, 232b ont été détachées des bords de la languette 200 et sont fixées sur le plan de la languette et de part et d'autre. Bien entendu l'invention concerne également la configuration symétrique, où seules les extrémités amont 231b, 232b des lamelles restent attachées aux bords de la languette et où ce sont les extrémités aval qui sont fixées sur le plan de la languette et de part et d'autre.

L'invention n'est pas limitée par la description qui précède :
- Le moyen d'introduction pourrait ne pas être plan et prendre une autre forme, par exemple celle d'un cylindre ou d'un fil,
- Le dispositif de prélèvement 300 pourrait comporter plusieurs dispositifs de capture, par exemple alignés longitudinalement ou en quinconce, de façon à ne pas trop perturber le flux sanguin ou transversalement sur la languette 200, les dispositifs de capture pouvant être différents deux à deux notamment en termes de forme, de taille, de dimension et de taille des ouvertures.

### EXEMPLES

### SIMULATION NUMERIQUE

### Matériel et Méthodes:

Afin d'évaluer la répercussion du dispositif de capture sur la vitesse du flux, le nombre de Reynolds, les contraintes de cisaillement et l'activation plaquettaire, l'influence sur le flux sanguin de différents prototypes de dispositif de capture a été simulée par « computational fluid dynamics » (CFD). Cette étape de simulation numérique de l'interaction fluide-structure (FSI) a été réalisée à l'aide du logiciel Ansys Fluent^{®} 15.0. Une étude bibliographique a permis de définir les constantes régissant l'écoulement du flux sanguin, ainsi que les algorithmes de calcul les plus appropriés.

Le sang est considéré comme un fluide non newtonien ce qui signifie que sa viscosité dépend du taux de cisaillement, contrairement aux fluides newtoniens, dont la viscosité est constante. La prise en compte de cette propriété est déterminante. Parmi les différents modèles pour simuler la viscosité des fluides non newtoniens, le modèle de la loi de puissance (Power Law Model) a été choisi (Petkova, S., et al.). Ce modèle s'écrit selon la formule suivante : ηₘᵢₙ > η = kγⁿ⁻¹e^{T0 T} < ηₘₐₓ ou k est une mesure de la viscosité moyenne du fluide (Indice de consistance), n est une mesure de la déviation du fluide newtonien (Indice de loi de puissance), T0 est la température de référence et ηmin et ηmax sont respectivement, les limites de viscosité inférieure et supérieure.Les paramètres de la loi de puissance utilisés lors des simulations sont présentés dans le tableau 1 ci-dessous.

**Tableau 1**

| | |
|---|---|
| Indice de la loi de puissance (n) | 0.4851 |
| Indice de consistance k (kg-s^n-2/m) | 0.2073 |
| Température de référence (°K) | 310 |
| Limite de viscosité supérieure ηₘₐₓ (kg/m-s) | 0.00125 |
| Limite de viscosité inférieure ηₘᵢₙ (kg/m-s) | 0.003 |

Le dispositif de capture étant préférentiellement destiné à être mis en place dans une veine de l'avant-bras, le modèle de veine utilisé a été celui de la veine basilique.

Un modèle de veine basilique a été développé sur la base de différentes études anatomiques dont Baptista-Silva et al..

La veine basilique a été modélisée en utilisant les variables présentées dans le tableau 2 ci-dessous (Munis, J.R., L.J. Bhatia S Fau - Lozada, and L.J. Lozada):

**Tableau 2**

| | |
|---|---|
| Diamètre de la veine (mm) | 2.0 |
| Vitesse du flux sanguin (m·s-1) | 0.0720 |
| Pression veineuse périphérique (mmHg) | 13 |

La thrombogénicité a été déterminée par l'étude des contraintes de cisaillement et du temps d'exposition, l'activation plaquettaire apparaissant à partir d'une valeur de 35 dyne·s/cm2 appelée seuil de Hellums (Bludszuweit, C.).

### Résultats :

### Détermination de la forme optimale du dispositif de capture :

Différentes formes de dispositif de capture ont été testées. Pour chacune de ces formes, l'influence du dispositif de capture sur la vitesse du flux, le nombre de Reynolds, les contraintes de cisaillement et l'activation plaquettaire du flux sanguin a été déterminée. Une cartographie 3D des vecteurs vitesse au sein et au voisinage du dispositif a été établie et ce pour différentes valeurs de la vitesse d'écoulement du sang au sein du canal.

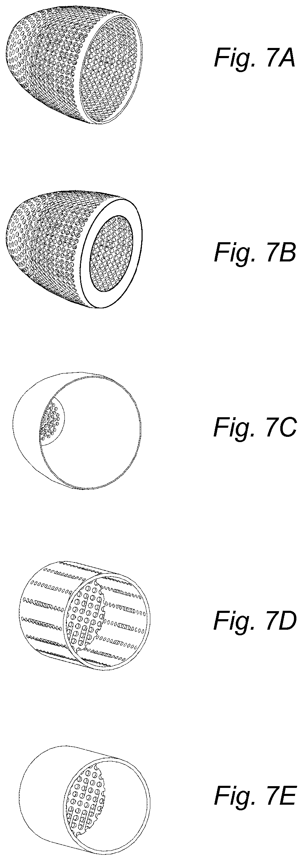

Les différentes formes testées sont illustrées aux figures 7A à 7E. Les résultats sont résumés dans le tableau 3.

**Tableau 3**

| Forme du dispositif de capture | Résultats |
|---|---|
| Figure 7A | -Conservation du caractère laminaire du flux sanguin (Reₘₐₓ=28) |
| Forme de type paraboloïde présentant des ouvertures sur l'intégralité du dispositif | -Présence de deux tourbillons contra-rotatifs à l'intérieur du dispositif de capture disparaissant à des vitesses de 0.2m/s |
| | -Diminution majeure de la vitesse du flux à l'intérieur du dispositif de capture |
| | -Absence d'accélération du flux au niveau des ouvertures |
| | - Augmentation progressive de la pression à l'intérieur du dispositif (valeur maximale de 1400 Pa au fond du dispositif) |
| | -Variation des contraintes de cisaillement entre 9.43 et 36.3 Pa |
| Figure 7B | -Conservation du caractère laminaire du flux sanguin |
| Forme de type paraboloïde présentant des ouvertures sur le corps du dispositif +anneau plein réduisant le diamètre de l'orifice central | -Présence de deux tourbillons contra-rotatifs au fond du dispositif de capture (reflux central) |
| Figure 7C | -Conservation du caractère laminaire du flux sanguin |
| Forme de type paraboloïde à fond plat présentant des ouvertures uniquement au | - Régime de pression d'emblée maximum dans le dispositif |
| | -Présence de deux tourbillons contra-rotatifs de petite taille à |
| niveau du fond plat | l'entrée du dispositif de capture |
| Figure 7D | -Conservation du caractère laminaire du flux sanguin |
| Forme de type cylindrique présentant des ouvertures sur l'intégralité du dispositif | -Diminution majeure de la vitesse du flux à l'intérieur du dispositif de capture |
| | -Absence d'accélération du flux au niveau des ouvertures |
| | - Augmentation très progressive de la pression à l'intérieur du dispositif (valeur maximale de 1380 Pa au fond du dispositif) |
| | -Présence de deux tourbillons contra-rotatifs à l'intérieur du dispositif de capture |
| Figure 7E | -Conservation du caractère laminaire du flux sanguin |
| Forme de type cylindrique présentant des ouvertures uniquement au niveau du fond du cylindre | - Pression d'emblée maximale à l'entrée du dispositif (valeur maximale de 1380 Pa à l'intérieur du dispositif) |
| | -Absence de tourbillons contra-rotatifs |

### Conclusions :

Les formes paraboloïdes et cylindriques permettent de conserver le caractère laminaire du flux sanguin.

La présence de tourbillons contra-rotatifs et de ce fait la recirculation des biomarqueurs est largement diminuée lorsque les parois du dispositif sont parallèles au flux et lorsque le dispositif de capture ne possède pas d'ouvertures sur ses parois latérales.

### EFFICACITE DE CAPTURE

### Matériels et méthodes

Les conditions de circulation dans une veine ont été reproduites in vitro.

L'échantillon de sang testé est un échantillon de sang de volontaire sain prélevé sur des tubes Vacutainer^{®} EDTA (Becton, Dickinson & Company), auquel des cellules tumorales PC3-GFP ont été ajoutées à une concentration de 500 à 25000 cellules tumorales/ml.

Des dispositifs de capture cylindrique tel que celui illustré à la figure 7B présentant différentes ouvertures ont été testés. Ces dispositifs possèdent des ouvertures avec un diamètre de 10µm pour les ouvertures cylindriques et de 15µm en entrée et 8µm en sortie pour les ouvertures coniques.

La viabilité des cellules capturées a ensuite été évaluées par retrait des cellules par trypsination et remises en culture de ces dernières dans des puits de culture.

### Résultats :

**Tableau 4**

| | | | | | |
|---|---|---|---|---|---|
| Longueur | 6µm | 150 µm | 150 µm | 150 µm | 150 µm |
| Epaisseur de la paroi | 6µm | 6µm | 6µm | 10µm | 10µm |
| Forme des ouvertures | cylindrique | cylindrique | conique | cylindrique | conique |
| Durée de l'expérience | 20min | 10min | 20min | 20min | 20min |
| Efficacité de capture | - | +++ | + | ++ | + |

### Conclusions :

Les résultats résumés dans le tableau 4 montrent que l'épaisseur et la longueur du dispositif de capture ont une influence sur l'efficacité de capture. Le dispositif de capture doit posséder une longueur_minimale donnée pour permettre la capture des biomarqueurs cellulaires. Sans cette longueur minimale les biomarqueurs cellulaires retenus par le moyen de filtration sont mis en recirculation quasi-immédiatement dans le flux biologique. L'épaisseur du dispositif de capture influe également l'efficacité de capture. Une épaisseur trop importante peut en effet diminuer l'efficacité de capture. Le dispositif de capture permet la capture spécifique de biomarqueurs cellulaires tels que les CTCs. Les autres cellules du sang telles que les leucocytes ne sont pas capturées. En effet, à la différence des CTCs, ces derniers possèdent des propriétés viscoélastiques qui leur permettent de passer au travers d'ouvertures inférieures à leur diamètre.

La forme circulaire des ouvertures est la mieux adaptée à la capture de biomarqueurs cellulaires tels que les CTCs. Le diamètre des ouvertures doit être adapté au diamètre des biomarqueurs, mais surtout au régime de pression rencontré dans le système veineux périphérique.

Les cellules cancéreuses capturées sont viables après capture.

### REFERENCES

Tout au long de cette demande, diverses références décrivent l'état de la technique à laquelle cette invention appartient. Les descriptions de ces références sont incorporées par référence dans la présente demande.
Baptista-Silva, J.C.C, et al. Anatomy of the basilic vein in the arm and its importance for surgery, Braz J. Morphol. Sci., 2003, 20(3): p.171-175
Bludszuweit, C., Three-dimensional numerical prédiction of stress loading of blood particles in a centrifugal pump. (0160-564X (Print)).
Hellums, J., et al., Studies on the mechanisms of shear-induced platelet activation, in Cérébral ischemia and hemorheology. 1987, Springer. p.80-89
Munis, J.R., L.J. Bhatia S Fau - Lozada, and L.J. Lozada, Peripheral venous pressure as a hemodynamic variable in neurosurgical patients. (0003-2999 (Print))
Nagrath, S., et al., Isolation of rare circulating tumour cells in cancer patients by microchip technology. Nature, 2007. 450 (7173): p.1235-9
Petkova, S., et al. CFD modelling of blood flow in portal vein hypertension with and without thrombosis. Third international conférence on CFD in the minerals and process industries. 2003
Small, A.C., et al., The emerging rôle of circulating tumor cell détection in genitourinary cancer. J Urol, 2012. 188(1): p.21-6
Williams, A., et al. Clinical translation of a novel microfilter technology Capture, characterization and culture of circulating tumor cells. Point-of-Care Healthcare Technologies (PHT), 2013 IEEE.

## Revendications

1. Dispositif de prélèvement (300) de biomarqueurs cellulaires, comportant :
- au moins un dispositif de capture (100) de biomarqueurs cellulaires, apte à être utilisé *in vivo* et comportant :
- un moyen de filtration (120) prévu pour retenir lesdits biomarqueurs cellulaires, ledit moyen de filtration comportant au moins une ouverture traversante (121) dont les dimensions sont adaptées pour retenir lesdits biomarqueurs cellulaires choisis dans le groupe consistant en des cellules stromales, épithéliales, des cellules foetales circulantes, des cellules souches, des clusters ou des agrégats de cellules tumorales, des cellules tumorales associées à tous types de cancer et les autres types cellulaires: monocytes, macrophages, et
- ledit moyen de filtration (120) est solidaire d'un support (110) prenant la forme d'une pièce creuse ouverte à une extrémité et fermée à une autre extrémité par le moyen de filtration, et possédant une symétrie de révolution autour d'un axe (X-X') totale ou tronquée selon un plan parallèle à l'axe (X-X'), le moyen de filtration formant le fond du support (110),
ledit dispositif de capture étant fixé sur un moyen d'introduction (200) dudit dispositif de capture dans un vaisseau de flux biologique.

2. Dispositif de prélèvement de biomarqueurs cellulaires selon la revendication 1, **caractérisé en ce que** l'au moins une ouverture traversante (121) présente au moins une dimension transversale comprise entre 1 µm et 200 µm, de préférence entre 5 µm et 25 µm.

3. Dispositif de prélèvement de biomarqueurs cellulaires selon l'une des revendications précédentes, **caractérisé en ce que** le moyen de filtration (120) est plan.

4. Dispositif de prélèvement de biomarqueurs cellulaires selon l'une des revendications précédentes, **caractérisé en ce que** le support (110) prend la forme d'un cylindre creux, tronqué selon un plan parallèle à l'axe (X-X') du cylindre, le moyen de filtration (120) formant le fond du cylindre tronqué.

5. Dispositif de prélèvement de biomarqueurs cellulaires selon l'une des revendications précédentes, **caractérisé en ce qu'**il est réalisé en une résine photosensible.

6. Dispositif de prélèvement de biomarqueurs cellulaires selon l'une des revendications précédentes, **caractérisé en ce que** le moyen d'introduction dudit dispositif de capture dans la circulation sanguine comporte une languette (200).

7. Dispositif de prélèvement de biomarqueurs cellulaires selon la revendication 6, **caractérisé en ce que** le moyen d'introduction (200) comporte un premier moyen de protection du moyen de filtration, prévu pour éviter le détachement du moyen de filtration.

8. Dispositif de prélèvement de biomarqueurs cellulaires selon la revendication 7, **caractérisé en ce que** le premier moyen de protection comporte au moins un plot (210) fixé sur le moyen d'introduction (200).

9. Dispositif de prélèvement de biomarqueurs cellulaires selon l'une des revendications précédentes, **caractérisé en ce que** le moyen d'introduction (200) comporte un second moyen de protection prévu pour protéger un vaisseau sanguin dans lequel il est amené à être introduit.

10. Dispositif de prélèvement de biomarqueurs cellulaires selon l'une des revendications 7 ou 9, **caractérisé en ce qu'**il comporte une pièce terminale (220) fixée à l'extrémité (201) du moyen d'introduction (200) et formant un premier et/ou un second moyen de protection.

11. Ensemble de prélèvement de biomarqueurs cellulaires comprenant un cathéter et un dispositif de prélèvement selon l'une des revendications précédentes, ledit dispositif étant inséré dans le cathéter.

12. Dispositif de prélèvement de biomarqueurs cellulaires selon l'une des revendications 1 à 10 pour une utilisation à des fins diagnostiques ou thérapeutiques.

13. Ensemble de prélèvement de biomarqueurs cellulaires selon la revendication 11 pour une utilisation à des fins diagnostiques ou thérapeutiques.

14. Dispositif de prélèvement de biomarqueurs cellulaires selon la revendication 12 ou ensemble de prélèvement de biomarqueurs cellulaires selon la revendication 13 caractérisé(s) en ce qu'il(s) sont destiné(s) à être utilisé(s) pour le diagnostic ou la thérapie du cancer.

## Patentansprüche

1. Vorrichtung zur Entnahme (300) von zellulären Biomarkern, umfassend:
- mindestens eine Vorrichtung zum Fangen (100) von zellulären Biomarkern, die *in vivo* verwendet werden kann und umfasst:
- ein Filtermittel (120), das dafür vorgesehen ist, die zellulären Biomarker zurückzuhalten, wobei das Filtermittel mindestens eine Durchgangsöffnung (121) umfasst, deren Abmessungen geeignet sind, um die zellulären Biomarker, ausgewählt aus der Gruppe bestehend aus Stroma-, Epithelzellen, zirkulierenden fötalen Zellen, Stammzellen, Clustern oder Aggregaten von Tumorzellen, Tumorzellen, die mit allen Arten von Krebs assoziiert sind, und den anderen Zelltypen: Monozyten, Makrophagen, zurückzuhalten, und
- das Filtermittel (120) fest mit einem Träger (110) verbunden ist, der die Form eines hohlen Stücks annimmt, das an einem Ende offen und an einem anderen Ende durch das Filtermittel geschlossen ist, und eine um eine Achse (X-X') vollständige, oder in einer Ebene parallel zur Achse (X-X') abgeschnittene Rotationssymmetrie besitzt, wobei das Filtermittel den Boden des Trägers (110) bildet,
wobei die Fangvorrichtung an einem Mittel zum Einführen (200) der Fangvorrichtung in ein Gefäß mit biologischem Fluss befestigt ist.

2. Vorrichtung zur Entnahme von zellulären Biomarkern nach Anspruch 1, **dadurch gekennzeichnet, dass** die mindestens eine Durchgangsöffnung (121) mindestens eine Querabmessung im Bereich zwischen 1 µm und 200 µm, vorzugsweise zwischen 5 µm und 25 µm, aufweist.

3. Vorrichtung zur Entnahme von zellulären Biomarkern nach einem der vorstehenden Ansprüche, **dadurch gekennzeichnet, dass** das Filtermittel (120) eben ist.

4. Vorrichtung zur Entnahme von zellulären Biomarkern nach einem der vorstehenden Ansprüche, **dadurch gekennzeichnet, dass** der Träger (110) die Form eines hohlen Zylinders annimmt, der in einer Ebene parallel zur Achse (X-X') des Zylinders abgeschnitten ist, wobei das Filtermittel (120) den Boden des abgeschnittenen Zylinders bildet.

5. Vorrichtung zur Entnahme von zellulären Biomarkern nach einem der vorstehenden Ansprüche, **dadurch gekennzeichnet, dass** sie aus einem lichtempfindlichen Harz hergestellt ist.

6. Vorrichtung zur Entnahme von zellulären Biomarkern nach einem der vorstehenden Ansprüche, **dadurch gekennzeichnet, dass** das Mittel zum Einführen der Fangvorrichtung in den Blutkreislauf eine Zunge (200) umfasst.

7. Vorrichtung zur Entnahme von zellulären Biomarkern nach Anspruch 6, **dadurch gekennzeichnet, dass** das Einführmittel (200) ein erstes Mittel zum Schutz des Filtermittels umfasst, das dafür vorgesehen ist, das Ablösen des Filtermittels zu verhindern.

8. Vorrichtung zur Entnahme von zellulären Biomarkern nach Anspruch 7, **dadurch gekennzeichnet, dass** das erste Schutzmittel mindestens einen Zapfen (210) umfasst, der am Einführmittel (200) befestigt ist.

9. Vorrichtung zur Entnahme von zellulären Biomarkern nach einem der vorstehenden Ansprüche, **dadurch gekennzeichnet, dass** das Einführmittel (200) ein zweites Schutzmittel umfasst, das dafür vorgesehen ist, ein Blutgefäß, in das sie eingeführt werden soll, zu schützen.

10. Vorrichtung zur Entnahme von zellulären Biomarkern nach einem der Ansprüche 7 oder 9, **dadurch gekennzeichnet, dass** sie ein Endstück (220) umfasst, das am Ende (201) des Einführmittels (200) befestigt ist und ein erstes und/oder ein zweites Schutzmittel bildet.

11. Set zur Entnahme von zellulären Biomarkern, umfassend einen Katheter und eine Entnahmevorrichtung nach einem der vorstehenden Ansprüche, wobei die Vorrichtung in den Katheter eingesetzt ist.

12. Vorrichtung zur Entnahme von zellulären Biomarkern nach einem der Ansprüche 1 bis 10 zur Verwendung für diagnostische oder therapeutische Zwecke.

13. Set zur Entnahme von zellulären Biomarkern nach Anspruch 11 zur Verwendung für diagnostische oder therapeutische Zwecke.

14. Vorrichtung zur Entnahme von zellulären Biomarkern nach Anspruch 12 oder Set zur Entnahme von zellulären Biomarkern nach Anspruch 13, **dadurch gekennzeichnet, dass** sie/es dazu bestimmt ist/sind, zur Diagnose oder Therapie von Krebs verwendet zu werden.

## Claims

1. Device (300) for sampling cellular biomarkers, comprising:
- at least one device (100) for capturing cellular biomarkers, suitable for use *in vivo* and comprising:
- filtration means (120) for retaining said cellular biomarkers, said filtration means comprising at least one through opening (121), the dimensions of which are adapted to retain said cellular biomarkers selected from the group consisting of stromal cells, epithelial cells, circulating foetal cells, stem cells, clusters or aggregates of tumour cells, tumour cells associated with all types of cancer and other cell types: monocytes, macrophages, and
- said filtration means (120) is integral with a support (110) taking the form of a hollow part open at one end and closed at the other end by the filtration means, and having a symmetry of revolution about an axis (X-X') which is totally or truncated in a plane parallel to the axis (X-X'), the filtration means forming the bottom of the support (110),
said capture device being attached to a means (200) for introducing said capture device into a biological flow vessel.

2. Device for sampling cellular biomarkers according to claim 1, **characterised in that** said at least one through opening (121) has at least one transverse dimension of between 1 µm and 200 µm, preferably between 5 µm and 25 µm.

3. Device for sampling cellular biomarkers according to one of the preceding claims, **characterised in that** the filtration means (120) is planar.

4. Device for sampling cellular biomarkers according to one of the preceding claims, **characterised in that** the support (110) takes the form of a hollow cylinder, truncated in a plane parallel to the axis (X-X') of the cylinder, the filtration means (120) forming the base of the truncated cylinder.

5. Device for sampling cellular biomarkers according to one of the preceding claims, **characterised in that** it is made of a photosensitive resin.

6. Device for sampling cellular biomarkers according to one of the preceding claims, **characterised in that** the means for introducing said capture device into the bloodstream comprises a tab (200).

7. Device for sampling cellular biomarkers according to claim 6, **characterised in that** the introduction means (200) comprises a first means for protecting the filtration means, designed to prevent the filtration means from becoming detached.

8. Device for sampling cellular biomarkers according to claim 7, **characterised in that** the first protection means comprises at least one plot (210) attached to the introduction means (200).

9. Device for sampling cellular biomarkers according to one of the preceding claims, **characterised in that** the introduction means (200) comprises a second protection means designed to protect a blood vessel into which it is to be introduced.

10. Device for sampling cellular biomarkers according to one of claims 7 or 9, **characterised in that** it comprises an end piece (220) attached to the end (201) of the introduction means (200) and forming a first and/or a second protection means.

11. Assembly for sampling cellular biomarkers comprising a catheter and a sampling device according to one of the preceding claims, said device being inserted into the catheter.

12. Device for sampling cellular biomarkers according to one of claims 1 to 10 for use for diagnostic or therapeutic purposes.

13. Assembly for sampling cellular biomarkers according to claim 11 for use in diagnosis or therapy.

14. Device for sampling cellular biomarkers according to claim 12 or assembly for sampling cellular biomarkers according to claim 13, **characterised in that** it/they is/are intended to be used for the diagnosis or therapy of cancer.
